# EUROPÄISCHE PATENTSCHRIFT

(11) **EP 0 548 640 B1**
(45) Veröffentlichungstag und Bekanntmachung des Hinweises auf die Patenterteilung: **12.07.2000**
(21) Anmeldenummer: 92120783.3
(22) Anmeldetag: 05.12.1992
(51) Int. Cl.: C07D 473/40, C07D 239/50

(54) **Verfahren zur Herstellung von 2,4,5-Triamino-6-halogenopyrimidinen und 2-Amino-6-halogenopurinen**
Process for the preparation of 2,4,5-triamino-6-halopyrimidines and 2-amino-6-halopurines
Procédé pour la préparation de 2,4,5-triamino-6-halogénopyrimidines et de 2-amino-6-halogénopurines

(30) Priorität: 21.12.1991 DE 4142568
(43) Veröffentlichungstag der Anmeldung: 30.06.1993
(73) Patentinhaber: BASF Aktiengesellschaft, 67063 Ludwigshafen (DE)
(72) Erfinder: Hagen, Helmut, Dr., W-6710 Frankenthal (DE); Raatz, Peter, Dr., W-6700 Ludwigshafen (DE)

(56) Entgegenhaltungen:
- EP-A- 0 335 355
- EP-A- 0 444 266
- US-A- 2 844 576
- JOURNAL OF MEDICINAL CHEMISTRY Bd. 15, Nr. 5, 1972, WASHINGTON Seiten 456 - 458 HOWARD J. SCHAEFFER ET. AL.
- JOURNAL OF MEDICINAL CHEMISTRY Bd. 17, Nr. 1, 1974, WASHINGTON Seiten 6 - 8 HOWARD SCHAEFFER ET. AL.
- JOURNAL OF THE AMERICAN CHEMICAL SOCIETY Bd. 80, Nr. 2, 25. Januar 1958, WASHINGTON Seiten 404 - 408 JOHN A. MONTGOMERY
- THE JOURNAL OF ORGANIC CHEMISTRY Bd. 25, Nr. 3, 11. April 1960, WASHINGTON Seiten 395 - 399 JOHN A. MONTGOMERY ET. AL.

## Beschreibung

Die vorliegende Erfindung betrifft ein neues Verfahren zur Herstellung von 2,4,5-Triamino-6-halogenopyrimidinen der Formel I 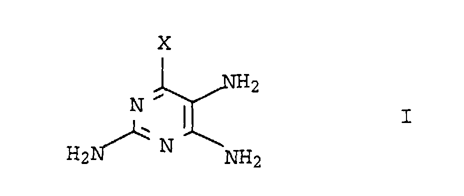 in der X Halogen bedeutet.

Außerdem betrifft die Erfindung ein neues Verfahren zur Herstellung von 2-Amino-6-halogenopurinen der Formel III

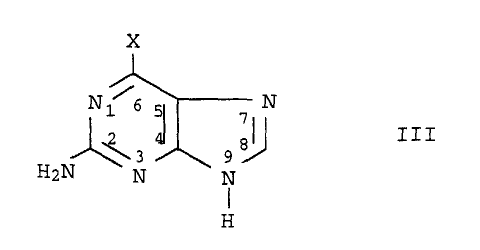

Pyrimidine, insbesondere 4,5-Diaminopyrimidine, sind allgemein bekannte Verbindungen und dienen als Ausgangssubstanzen für die Synthese von Purinen, welche ihrerseits für die Herstellung von Pharmazeutika von Bedeutung sind. Aufgrund ihrer Antitumorwirkung sind besonders die Halogenopurine von allgemeinem Interesse. Sie dienen als wertvolle Zwischenprodukte für die Synthese von Purinnucleosiden.

Aus der EP-A 335 355 ist es bekannt, 2,4,5-Triamino-6-chlorpyrimidin durch Hydrogenolyse von 2,4-Diamino-5-[4-chlorphenyl)-azo]-6-chlorpyrimidin in Gegenwart von überstöchiometrischen Mengen Zinkstaub herzustellen. Da bei diesem Verfahren Feststoffe als Reaktionspartner beteiligt sind, ist es für technische Synthesen wenig geeignet.

In J. Org. Chem. 19, 930 (1954) wird die Herstellung von 2,4,6-Trichlor-5-aminopyrimidin durch katalytische Hydrierung der entsprechenden 5-Nitroverbindung an Raney-Nickel beschrieben.

Ferner ist die Hydrierung von 4-Amino-5-nitro-6-propylaminopyrimidin zu der entsprechenden 4,5-Diaminoverbindung mit Hilfe von Palladium auf Aktivkohle bekannt (Biochemistry 10, 3338 (1971)), und außerdem wurde auch bereits 2,6-Dihydroxy-4-chlor-5-nitropyrimidin an Platin auf Aktivkohle hydriert (J. Med. Chem. 11, 1107 (1968)).

Seit langem ist die durch Umsetzung von o-Phenylendiamin mit Ameisensäure durchführbare Cyclisierungsreaktion zum Benzimidazol bekannt. Als Methode zur Cyclisierung von 4,5-Diaminopyrimidin und seiner Derivate zu den entsprechenden Purinen ist der Einbau von C₁-Bausteinen auf der Basis von Ameisensäurederivaten bekannt.

So beschreiben Montgomery et al. (J. Am. Chem. Soc. 80, 404 (1958)) die Umsetzung von 2,6-Dichlor-4,5-diaminopyrimidin mit Essigsäurediethoxymethylester zum 2,6-Dichlorpurin bei Temperaturen von 100 bis 120°C.

Ferner werden in J. Org. Chem. 25, 395 (1960) Reaktionen von 4,5-Diaminochlorpyrimidinen mit Orthoestern zu den entsprechenden Chlorpurinen beschrieben.

US 2,844,576 lehrt die Herstellung von 2-Amino-6-halogenpurinen durch Cyclisierung der entsprechenden 2,4,5-Triamino-6-halogenpyrimidine in Gegenwart von Alkylorthoformiaten und Alkanoylanhydriden.

Aus Scheaffer et al., J. Med. Chem. 1972, 15, 456-458 und 1974, 17, 6-8 ist die Herstellung von an Position 9 substituierten Adeninen durch Cyclisierung der entsprechend substituierten Amine mit Hilfe von Triethylorthoformiaten bekannt.

Eine der vorliegenden Erfindung zugrunde liegende Aufgabe war es, den geschilderten Mängeln abzuhelfen und ein technisch brauchbares Verfahren zur Herstellung von 2,4,5-Triamino-6-halogenopyrimidinen (I) bereitzustellen, um diese für die Synthese von 2-Amino-6-halogenopurinen (III) wichtigen Zwischenprodukte einfacher und wirtschaftlicher zugänglich zu machen. Besonders im Hinblick auf die Herstellung von 2-Amino-6-halogenopurinen (III) lag der Erfindung als weitere Aufgabe zugrunde, ein einfaches Verfahren zur Cyclisierung von (I) mit einem Ameisensäurederivat IVa, IVb oder IVc zur Verfügung zu stellen.

Demgemäß wurde ein Verfahren zur Herstellung von 2,4,5-Triamino-6-halogenopyrimidinen der Formel I 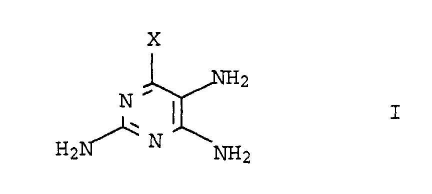 in der X Halogen bedeutet, gefunden, welches dadurch gekennzeichnet ist, daß man 2,4-Diamino-5-nitro-6-halogenopyrimidine der Formel II 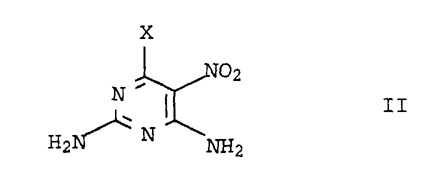 katalytisch mit Wasserstoff in Gegenwart eines festen Hydrierkatalysators in flüssigem Medium hydriert.

Weiterhin wurde ein Verfahren zur Herstellung von 2-Amino-6-halogenopurinen der Formel III 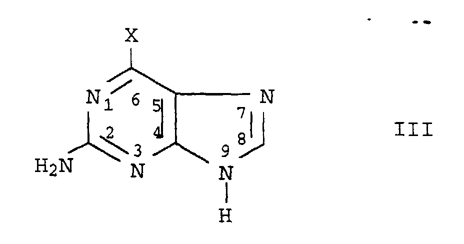 gefunden, welches dadurch gekennzeichnet ist, daß man ein Pyrimidin I mit einem Ameisensäurederivat der Formel IVa oder IVb

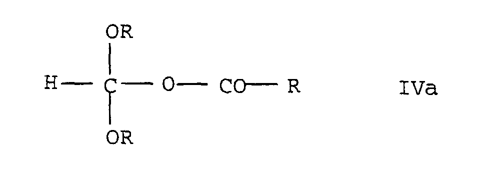

H―C(OR)_{3-Y}(NR₂)_{Y} IVb

umsetzt, wobei R gleiche oder verschiedene C₁-C₆-Alkylgruppen und Y 0 bis 3 bedeuten.

Als 2,4-Diamino-5-nitro-6-halogenopyrimidine II kommen neben den in 6-Stellung durch Fluor, Brom oder Jod substituierten Verbindungen insbesondere 2, 4-Diamino-5-nitro-6-chlorpyrimidin in Betracht, welches durch Nitrierung von 2,4-Diamino-6-chlorpyrimidin erhältlich ist (J. Med. Chem. 9, 573 (1966)).

Als Katalysatoren für die Hydrierung der 2,4-Diamino-5-nitro-6-halogenopyrimidine II eignen sich vor allem Oxide und Oxidhydrate der Edelmetalle Platin und Palladium, wobei Platindioxid besonders geeignet ist. Weiterhin kommen Raney-Nickel und die genannten Edelmetalle in Form von Trägerkatalysatoren in Betracht. Beispielsweise seien Platin/Aktivkohle- und Palladium/Aktivkohle-Katalysatoren mit etwa 1 bis 10 Gew.-% Metallgehalt genannt.

Die Menge des Katalysators liegt im allgemeinen zwischen 1 und 1000 g, vorzugsweise zwischen 50 und 100 g Metall pro kg II.

Die Hydriertemperatur kann in einem Bereich von etwa 0°C bis ungefähr zum Siedepunkt des flüssigen Mediums liegen. Vorzugsweise nimmt man aber die Hyrierung bei 10 bis 35°C vor, da sich unterhalb von 10°C die Reaktion zu sehr verlangsamt.

Für den Wasserstoffpartialdruck kommt ein Bereich von etwa 1 bis 100 bar in Betracht, wobei sich für praktische Zwecke der Bereich von Normaldruck bis etwa 10 bar besonders empfiehlt.

Als Reaktionsmedium nimmt man vorzugsweise Flüssigkeiten, in denen sich die Edukte lösen, teilweise lösen oder in suspendierter Form vorliegen. Beispielsweise kommen als Flüssigkeiten Alkanole wie Methanol, Ethanol und Isopropanol, mehrwertige Alkohole wie Ethylenglykol und Diethylenglykol, Ether wie Ethylenglykolmonoalkylether, Ethylenglykoldialkylether, Tetrahydrofuran und 1,4-Dioxan sowie daneben auch Acetonitril, N,N-Dimethylformamid und N-Methylpyrrolidon in Betracht. Man kann auch solche Systeme wählen, in denen sie sich nur teilweise lösen, z.B. Kohlenwasserstoffe.

Besonders empfiehlt sich die Verwendung von tiefsiedenden Flüssigkeiten wie Methanol und Ethanol, da diese vom Umsetzungsgemisch leicht abgetrennt werden können. Aufgrund seines Lösungsverhaltens ist insbesondere Ethanol bevorzugt.

In der Regel verwendet man eine Menge von 5 bis 100 kg Flüssigkeit pro kg II.

Beim Hydrierverfahren kann man wie üblich vorgehen. Zuerst erfolgt eine Vorbehandlung des Katalysators, indem man ihn bei Raumtemperatur im flüssigen Medium suspendiert und so lange Wasserstoff einleitet, bis kein weiterer aufgenommen wird. Anschließend wird die Umsetzung und Aufarbeitung des Reaktionsgemisches nach den üblichen Arbeitstechniken vorgenommen.

Das erfindungsgemäße Hydrierverfahren zur Herstellung von 2,4,5-Triamino-6-halogenopyrimidinen (I) weist gegenüber dem Stand der Technik den Vorteil auf, daß das Verfahrensprodukt (I) auf einfache Weise zu isolieren ist und der Hydrierkontakt nach seiner Regenierung wieder verwendet werden kann, womit sich das Verfahren auch für die Synthese von größeren Produktmengen im technischen Maßstab eignet. Ferner erfolgt die Hydrierung zum gewünschten 5-Amino-6-halogenopyrimidin (I) selektiv; Nebenreaktionen wie die Dehalogenierung der Ausgangssubstanz (II) konnten nicht beobachtet werden.

2,4,5-Triamino-6-halogenopyrimidine (I) sind wertvolle Zwischenprodukte für organische Synthesen, u.a. zur Herstellung von Arzneimitteln, welche auf der Substanzklasse der Purine aufbauen.

Besondere Bedeutung haben hierbei die Purine III, die nach dem oben genannten Verfahren durch Cyclisierung mit einem C₁-Baustein sehr vorteilhaft erhältlich sind.

Als Verbindung IVa kommen vorzugsweise Dimethoxy- und Diethoxymethylester von C₂-C₆-Carbonsäuren in Betracht, wie beispielsweise Essigsäuredimethoxymethylester und Essigsäurediethoxymethylester.

Als Verbindung IVb eignen sich Orthoameisensäuretrimethylester, Orthoameisensäuretriethylester sowie N,N-Dimethylformamiddimethylacetal.

Unter den Ameisensäurederivaten ist neben N,N-Dimethylformamiddimethylacetal Essigsäurediethoxymethylester als C₁-Baustein besonders bevorzugt.

Im allgemeinen setzt man die Verbindung IVa bzw. IVb in geringen Überschuß über die stöchiometrisch erforderliche Menge ein, wobei bevorzugt je Mol Pyrimidin I vorteilhafterweise 1 bis 3 mol, insbesondere 1 bis 1,3 mol des C₁-Bausteins verwendet werden. Bei größeren Überschüssen an Verbindung IVa, IVb oder IVc ist mit der Bildung von 2-Formylamino-6-halogenopurin als Nebenprodukt zu rechnen.

Da bei der Umsetzung aus dem Ameisensäurederivat IVa Carbonsäuren freigesetzt werden, die eine hydrolytische Abspaltung des 6-Halogenatoms bewirken können, empfiehlt es sich, bei einem pH-Wert von etwa 6 bis 8 zu arbeiten, der in üblicher Weise durch Puffersysteme wie Alkalisalze von Carbonsäuren konstant gehalten werden kann.

Bei Verwendung der Verbindung IVb sind zusätzliche katalytische Mengen einer Mineralsäure wie Salzsäure, Schwefelsäure und Salpetersäure oder aliphatische Carbonsäuren wie Essigsäure und Propionsäure zu empfehlen.

Als Lösungsmittel kommen Ether wie Ethylenglykoldialkylether, Tetrahydrofuran und 1,4-Dioxan sowie Acetonitril, N,N-Dimethylformamid, N,N-Dimethylacetamid und N-Methylpyrrolidon in Betracht, wobei N-Methylpyrrolidon besonders bevorzugt ist.

Für die Aufarbeitung des Reaktionsgemisches werden die üblichen Methoden wie Destillation, Filtration und Digerieren von Feststoffen in Lösungsmitteln herangezogen.

6-Halogenopurine (III), die nach dem erfindungsgemäßen Verfahren hergestellt werden, sind wertvolle Produkte für den Arzneimittelsektor.

Besondere Bedeutung haben das erfindungsgemäße Hydrier- und Cyclisierungsverfahren für die Herstellung von 2-Amino-6-chlorpurin aus 2,4,5-Triamino-6-chlorpyrimidin und Essigsäurediethoxymethylester, wobei die Herstellung der Pyrimidinverbindung durch Hydrierung von 2,4-Diamino-5-nitro-6-chlorpyrimidin erfolgt.

### Beispiel 1

### Herstellung von 2,4,5-Triamino-6-chlorpyrimidin

Nach der Behandlung einer Suspension aus 1 g Platindioxidhydrat in 200 ml Ethanol mit Wasserstoff wurden 9,5 g (50 mmol) 2,4-Diamino-5-nitro-6-chlorpyrimidin 4 Stunden bei Raumtemperatur bei einem Druck von 1 bar Wasserstoff hydriert. Anschließend wurde der Katalysator unter Stickstoff als Schutzgas abfiltriert und mit 50 ml Ethanol extrahiert.

Die übliche Aufarbeitung von Filtrat und Extrakt lieferte die obengenannte Verbindung in 82%iger Ausbeute.

### Beispiel 2

### Herstellung von 2-Amino-6-chlorpurin

Unter Stickstoff wurden 6,5 g (41 mmol) 2,4,5-Triamino-6-chlorpyrimidin in 100 ml N-Methylpyrrolidon gelöst und mit 8,1 g (50 mmol) Essigsäurediethoxymethylester versetzt. Während 4-ständigen Rührens bei 120°C wurden die Nebenprodukte Essigsäure und Methanol destillativ entfernt. Anschließend wurde das Rohprodukt bei 0°C auskristallisiert und durch Säulenchromatographie an Kieselgel (Laufmittel: Essigester/Methanol = 2/1) gereinigt.

Ausbeute: 63 %

## Patentansprüche

1. Verfahren zur Herstellung von 2-Amino-6-halogenopurinen der Formel III 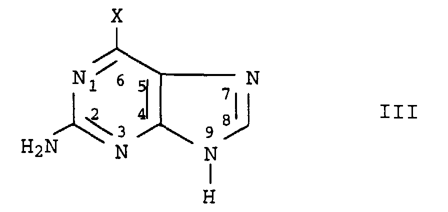 dadurch gekennzeichnet, daß man 2,4,5-Triamino-6-halogenopyrimidine der Formel I 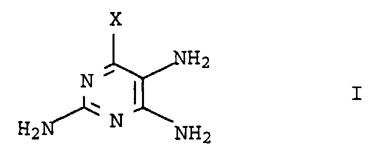 in der X Halogen bedeutet
mit einem Ameisensäurederivat der Formel IVa oder IVb 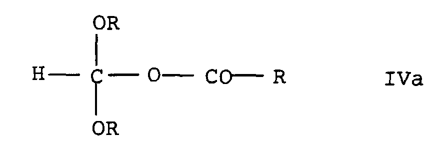
H―C(OR)_{3-Y}(NR₂)_{Y} IVb
umsetzt, wobei R gleiche oder verschiedene C₁-C₆-Alkylgruppen und Y 0 bis 3 bedeuten.

2. Verfahren zur Herstellung von 2-Amino-6-halogenopurinen der Formel III 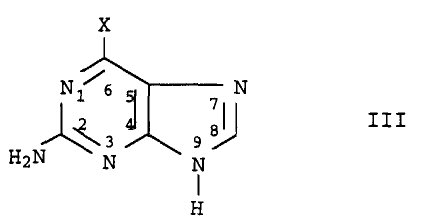 dadurch gekennzeichnet, daß man 2,4,5-Triamino-6-halogenopyrimidine der Formel I 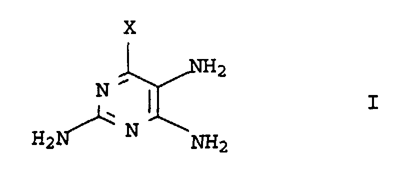 in der X Halogen bedeutet
mit einem Ameisensäurederivat der Formel IVa oder IVb 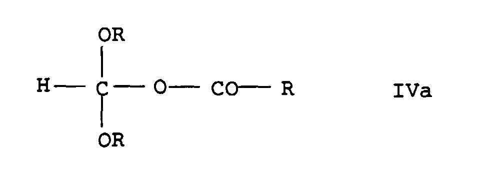
H―C(OR)_{3-Y}(NR₂)_{Y} IVb
umsetzt, wobei R gleiche oder verschiedene C₁-C₆-Alkylgruppen und Y 0 bis 3 bedeuten und
die Pyrimidine der Formel I
durch ein Verfahren hergestellt werden, bei dem 2,4-Diamino-5-nitro-6-halogenopyrimidine der Formel II 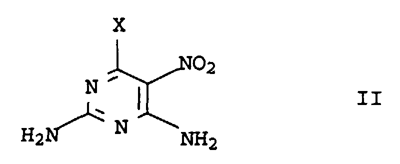 katalytisch mit Wasserstoff in Gegenwart eines festen Hydrierkatalysators in flüssigem Medium hydriert werden.

## Claims

1. A process for preparing 2-amino-6-halopurines of the formula III 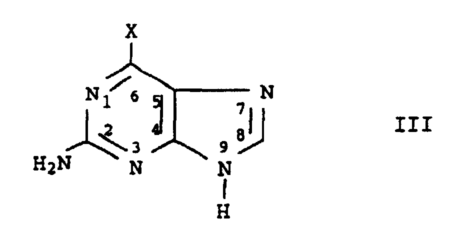 which comprises reacting 2,4,5-triamino-6-halopyrimidines of the formula I 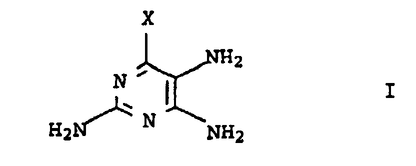 where X is halogen,
with a formic acid derivative of the formula IVa or IVb 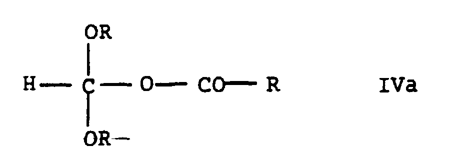
H―C(OR)_{3-Y}(NR₂)_{Y} IVb
where R are identical or different C₁-C₆-alkyl groups and Y is from 0 to 3.

2. A process for preparing 2-amino-6-halopurines of the formula III 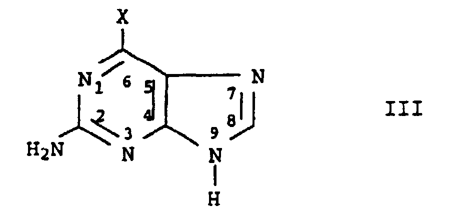 which comprises reacting 2,4,5-triamino-6-halopyrimidines of the formula I 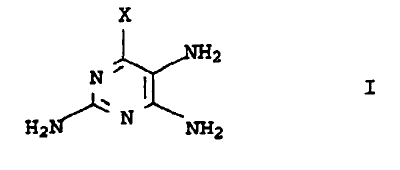 where X is halogen,
with a formic acid derivative of the formula IVa or IVb 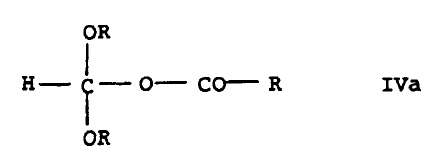
H―C (OR)_{3-Y} (Na₂)_{Y} IVb
where R are identical or different C₁-C₆-alkyl groups and Y is from 0 to 3, and
the pyrimidines of the formula I are prepared by a process in which 2,4-diamino-5-nitro-6-halopyrimidines of the formula II 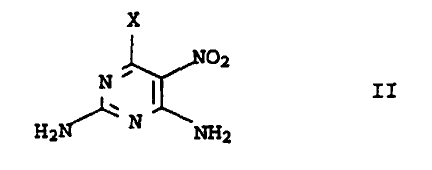 are catalytically hydrogenated in a liquid medium by means of hydrogen in the presence of a solid hydrogenation catalyst.

## Revendications

1. Procédé pour la préparation de 2-amino-6-halogénopurines de formule III 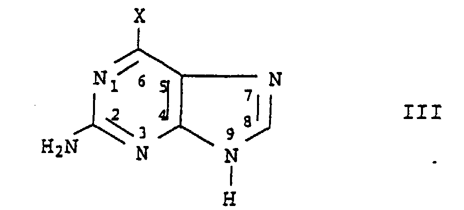 caractérisé par le fait que l'on fait réagir des 2,4,5-triamino-6-halogénopyrimidines de formule I 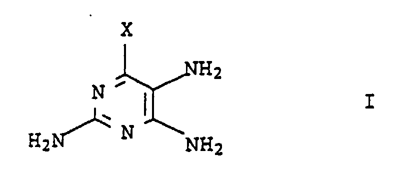 dans laquelle X représente un halogène,
avec un dérivé de l'acide formique de formule IVa ou IVb 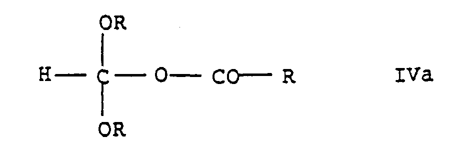
H―C(OR)_{3-Y}(NR₂)_{Y} IVb
les symboles R, ayant des significations identiques ou différentes, représentant des groupes alkyle en C1-C6 et Y un nombre allant de 0 à 3.

2. Procédé pour la préparation de 2-amino-6-halogénopurines de formule III 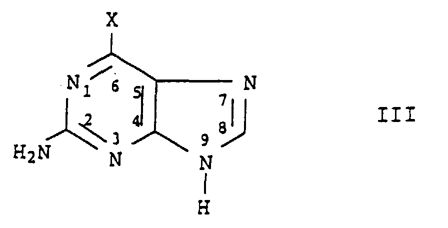 caractérisé par le fait que l'on fait réagir des 2,4,5-triamino-6-halogénopyrimidines de formule I 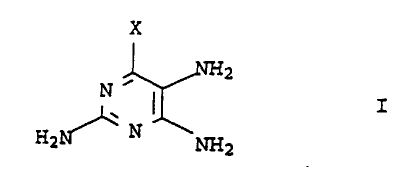 dans laquelle X représente un halogène,
avec un dérivé de l'acide formique de formule IVa ou IVb 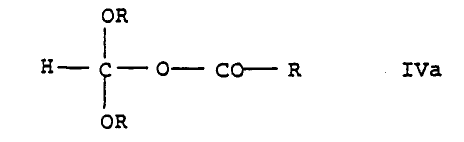
H―C(OR)_{3-Y}(NR₂)_{Y} IVb
les symboles R, ayant des significations identiques ou différentes, représentant des groupes alkyle en C1-C6 et Y un nombre allant de 0 à 3 et
les pyrimidines de formule I étant elles-mêmes préparées par un procédé dans lequel on soumet les 2,4-diamino-5-nitro-6-halogénopyrimidines de formule II 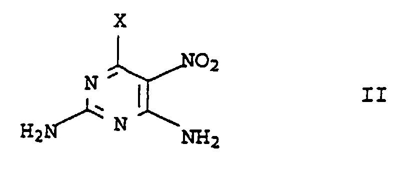 à hydrogénation catalytique en présence d'un catalyseur d'hydrogénation solide en milieu liquide.
